# EUROPEAN PATENT APPLICATION

(11) **EP 1 843 150 A1**
(43) Date of publication of application: **10.10.2007**
(21) Application number: 06712182.2
(22) Date of filing: 23.01.2006
(51) Int. Cl.: G01N 27/327, G01N 35/02

(54) **CONCATENATED BODY OF SENSOR CHIPS AND MANUFACTURING METHOD THEREOF**

(30) Priority: 24.01.2005 JP 2005015644
(71) Applicant: Sumitomo Electric Industries, Ltd., Osaka-shi, Osaka 541-0041 (JP); National Institute of Advanced Industrial Science and Technology, Tokyo 100-8921 (JP)
(72) Inventor: KAIMORI, Shingo Osaka W. of Sumitomo E.Ind. Ltd., Osaka-shi, Osaka 554-0024 (JP); HOSOYA, Toshifumi Osaka W. of Sumitomo E.Ind. Ltd., Osaka-shi, Osaka 554-0024 (JP); ICHINO, MoriyasuOsaka W. of Sumitomo E.Ind. Ltd., Osaka-shi, Osaka 554-0024 (JP); NAKAMURA, Hideaki Nat.Inst. of Adv.Ind. S.&Tech., Tsukuba-shi, Ibarak 305-8562 (JP); GOTOH, Masao Nat.Inst. of Adv.Ind. S.&Tech., Tsukuba-shi, Ibarak 305-8562 (JP); KURUSU, Fumiyo Nat.Inst. of Adv.Ind. S.&Tech., Tsukuba-shi, Ibarak 305-8562 (JP); ISHIKAWA, Tomoko Nat.Inst. of Adv.Ind. S.&Tech., Tsukuba-shi, Ibarak 305-8562 (JP); KARUBE, Isao Nat.Inst. of Adv.Ind. S.&Tech., Tsukuba-shi, Ibarak 305-8562 (JP)
(74) Representative: Cross, Rupert Edward Blount
(86) International application number: PCT/JP2006/300966
(87) International publication number: WO 2006/078016

(57) **Abstract**

A coupled member made of a plurality of sensor chips is featured by that adjacent sensor chips are coupled to each other under cuttable condition; the respective sensor chips can be cut and piece-separated from the coupled member in a higher efficiency; and even after the sensor chips are piece-separated, these sensor chips can be readily and quickly stored in containers thereof; and furthermore, the sensor chips can be easily checked and failure chips can be readily removed. More specifically, a method formanufacturinga coupled member made of the plurality of sensor chips is provided in which such sensor chips are coupled to each other under cuttable conditions, while each of these sensor chips includes: a base plate; a cover layer; a hollow reaction portion provided between the base plate and the cover layer; a sensing member provided in the hollow reaction portion; an output terminal for outputting a signal sensed by the sensing member to the outside; and a sample introducing port for introducing a sample to the hollow reaction portion.

## Description

### Technical Field

The present invention is related to a sensor chip capable of simply detecting and of quantitative-analyzing a chemical substance contained in a sample. More specifically, the present invention is directed to a coupled member made of such sensor chips that a plurality of biosensor chips are coupled to each other under cuttable condition, and also, directed to a method of manufacturing the sensor chip coupled member.

### Background Art

Biosensor chips correspond to such sensor chips that very small amounts of samples are introduced to reaction portions formed in these sensor chips; biochemical reactions such as enzyme reactions, antigen-antibody reactions, and the like may occur with respect to these very small amounts of the introduced samples within the sensor chips, and then, information acquired from the biochemical reactions is outputted from the sensor chips. These biosensor chips are utilized as, for example, blood sugar level sensors and urinary sugar level sensors in order to perform home health diagnoses (self-medical cares) capable of self-managing and preventing diabetes. The blood sugar level sensors measure glucose amounts (blood sugar levels) in blood and the urinary sugar level sensors measure glucose amounts in urine.

JP-A-2001-159618 (patent publication 1) discloses one example of such biosensor chips. This biosensor chip is equipped with a cavity (hollow reaction portion) into which a fluid sample is introduced due to capillary phenomenon. In this sensor chip (Claim 1), the introduced fluid sample is reacted with a reagent so as to analyze components contained in the fluid sample, and the biosensor chip is provided with an electrode system (sensing member) constituted by an operation electrode and a counter electrode, a lead (output terminals) for outputting sensed signal to an external unit, and a sample introducing port in addition to the hollow reaction portion.

When such sensor chips are manufactured for each sheet of these sensor chips (unit of one sheet), productivity thereof is lowered, and distances between base plates and cover layers and dimensions of hollow reaction portions are easily fluctuated for each of these sensor chips, and thus, measurement values are fluctuated, resulting in problems. As a consequence, the following methods may be preferably conceived. That is, while a large sheet is employed in order to form base plates and cover layers with respect to a large number of sensor chips, large sets of sensing member and hollow reaction portions are formed in a parallel manner on one sheet of the large sheet, so that a coupled member constituted by a large number of sensor chips is constructed. Thereafter, the sensor chips are individually cut out.

As a method for cutting off (piece-separating) a coupled member to individual sensor chips, such a method for cutting off a large number of sensor chips in a batch manner by a press process, or the like may be employed. Since cutting of these sensor chips is carried out in a batch manner, the manufacturing efficiency for piece-separating these sensor chips is high. However, in order to ship the sensor chips as products, a large number of sensor chips must be sorted out to be stored in containers. A lengthy time is necessarily required in order to store these sensor chips into the containers. As a result, the manufacturing efficiency which covers a series of manufacturing steps involving the container storages is eventually lowered.

Also, before sensor chips are shipped as products, each of these sensor chips must be examined so as to remove failure sensor chips. If the coupled member is piece-separated and thereafter the respective sensor chips are checked, then these individual sensor chips can be hardly transported to checking apparatuses, and such a cumbersome work is necessarily required in order to sort out these sensor chips and load the sorted sensor chips on the checking apparatuses. As a result, the failure sensor chips can be hardly removed.

As a method for piece-separating sensor chips, the below-mentioned method may be conceived. That is, respective sensor chips are cut out one by one from a coupled member made of a large number of sensor chips just before the individual sensor chips are stored in containers so as to be examined. In accordance with this method, the sensor chips can be easily stored in the containers and the failure chips can be readily removed. However, although the sensor chips can be quickly cut out in a relatively simple manner, a lengthy time is required in order to determine precise cutting positions of these chips. As a result, there is another problem that the efficiency of the piece-separating operation is lowered.
Patent Publication 1: JP-A-2001-159618 (claim 1)

### Disclosure of the Invention

### Problems to be solved by the Invention

The present invention is made to solve the problems of the conventional techniques, and has an object to provide a coupled member made of a plurality of sensor chips, and a manufacturing method thereof, by which in the coupled number of sensor chips where a large number of sensor chips are coupled to each other, the respective sensor chips can be cut and piece-separated from the coupled member in a higher efficiency; and even after the sensor chips are piece-separated, these sensor chips can be readily and quickly stored in containers thereof; and furthermore, the sensor chips can be easily checked and failure chips can be readily removed.

### Means for solving the Problems

The inventors of the present invention have deeply considered in order to solve the above-described problems, and as a result, find out that such a coupled member made of a plurality of sensor chips can achieve the above-mentioned object, in which respective sensor chips for constructing the coupled member of the sensor chips are coupled to each other by an easily cuttable means. Then, the inventors accomplish the present invention arranged by the below-mentioned structures.

According to Claim 1 of the present invention, there is provided a coupled member made of a plurality of sensor chips, wherein adjacent sensor chips are coupled to each other under cuttable condition.

In this case, such a condition that adjacent sensor chips are coupled to each other under cuttable condition means that these sensor chips are directly coupled to each other, or are coupled via other members to each other in such a manner that these sensor chips can be cut off so as to be formed as individual sensor chips by applying weak force (cutting off force) to these sensor chips, while this weak force never tears up, and also never cuts other portions.

In accordance with the present invention having the above-described structure, since the cutting off force is applied to predetermined spaces among these sensor chips, sensor chips can be cut off one by one from the coupled member where a large number of sensor chips are coupled to each other. Since the positions to be cut off are limited only to such coupling portions where the adjacent sensor chips are coupled to each other under cuttable condition, there is no longer required to determine such a cutting position. As a consequence, while there is no longer required to take time for determining the cutting positions, a higher piece-separating efficiency can be achieved. Also, in this method, the sensor chips are cut off one by one just before these individual sensor chips are stored in the containers, the chip storing operations may be readily carried out, and the failure chips may be easily removed.

Furthermore, the coupled member made of the sensor chips having the above-described structures may be stored in a container by being folded, or rounded without being cut off, and then, the coupled member stored in the container may be provided to consumers. In this case, these consumers may piece-separate the coupled member so as to obtain individual sensor chips when the consumers use these sensor chips. In such a case that piece-separated sensor chips are stored in a container and then the individual sensor chips stored in this container are shipped, when a consumer picks up one necessary chip from the container in order to use sensor chips, there are many cases that all of these stored sensor chips are dropped from the container to be dispersed, and there is a great possibility that some chips are lost. However, if the above-described coupled member made of the plurality of sensor chips is directly stored in the container, then this risk can be removed, so that the coupled member can be easily handled, resulting in a preferable result.

The present invention provides inventive ideas constructed of the below-mentioned structures as more preferred embodiment modes related to Claim 1 of the present invention.

There is provided the coupled member made of a plurality of sensor chips as in Claim 1, wherein
the sensor chip including:
a base plate;
a cover layer;
a hollow reaction portion provided between the base plate and the cover layer;
a sensing member provided in the hollow reaction portion;
an output terminal for outputting a signal sensed by the sensing member to the outside; and
a sample introducing port for introducing a sample to the hollow reaction portion (Claim 2).

Normally, the sensor chip employed as a biosensor chip and the like includes: the base plate; the cover layer; the hollow reaction portion provided between the base plate and the cover layer; the sensingmember provided in the hollow reaction portion; the output terminal for outputting the signal sensed by the sensing member to the outside; and the sample introducing port for introducing the sample to the hollow reaction portion. Claim 2 corresponds to this embodiment mode.

There is the coupled member made of a plurality of sensor chips as in the above-described coupled member, wherein
the adjacent sensor chips are directly coupled to each other by a weak coupling force portion located between the sensor chips (Claim 3).

In this case, the weak coupling force portion corresponds to such a portion which is located between adjacent sensor chips and which directly couples these adjacent sensor chips to each other under cuttable condition. Directly coupling of these adjacent sensor chips means that these sensor chips are coupled to each other without via any member. Since any member is not employed, after the coupled member is piece-separated, there is no disposed portion, but also this weak coupling force portion constitutes a part of a sensor chip. In such a case where the adjacent sensor chips are coupled to each other by the weak coupling force portion, it is preferable that these adjacent sensor chips in this coupling portion are contacted to each other in a line contact manner, or are located in the vicinity of each other.

There is the coupled member made of a plurality of sensor chips as in the above-described the coupled member, wherein
the adjacent sensor chips are coupled to each other via a holding body (Claim 4).

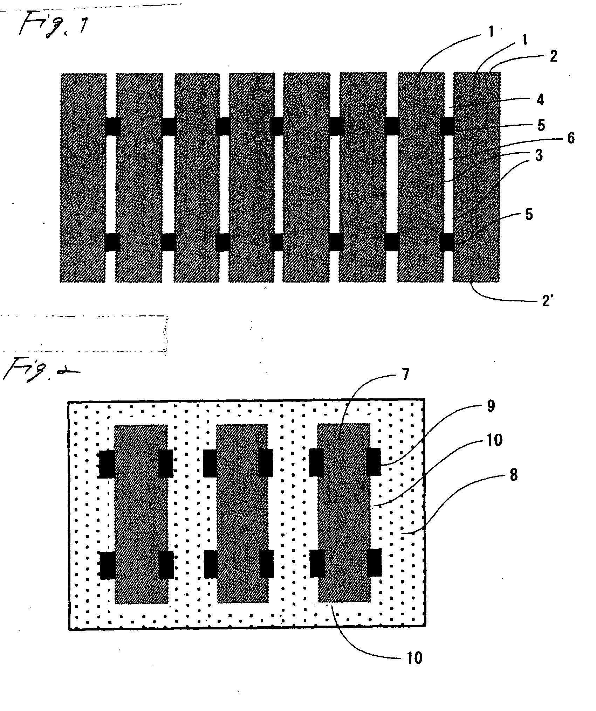

As methods for coupling the adjacent sensor chips, in addition to the method for directly coupling these adjacent sensor chips by the weak coupling force portion, as indicating in Fig. 2, there is another method that a plurality of sensor chips are coupled to each other via a holding body, and are arrayed to be held.

In this case, although the holding body is employed so as to couple the sensor chips to each other, this holding body does not constitute the sensor chips. As a consequence, after the sensor chips are cut off, this holding body is disposed. As a method for manufacturing the coupled member made of the sensor chips in which the sensor chips are coupled to each other via the holding body, the below-mentioned manufacturing method may be conceived. That is, for example, a plurality of sensor chips are formed by making intervals therebetween by employing a large sheet which forms a plurality of base plates and a plurality of cover layers, and thereafter, such a weak coupling force portion similar to the above-described weak coupling force portion is formed between a portion where the sensor chips are formed and a portion of the interval. In this case, this interval portion constitutes the holding body, so that the adjacent sensor chips are coupled via this portion to each other. Namely, such a portion of the employed large sheet where no sensor chip is formed constitutes the holding body.

As the holding body, another sheet other than the sheet employed in order to form the sensor chips may be alternatively employed. In accordance with the method for coupling the sensor chips by employing the holding body, after the sensor chips are cut off, the holding body must be disposed. As a consequence, such a method for providing the weak coupling force portion between the adjacent sensor chips so as to directly couple these adj acent sensor chips with each other is preferable, since such a cumbersome work for removing and disposing the holding body can be eliminated.

As materials for forming the weak coupling force portion, for example, an adhesive tape for connecting adjacent sensor chips may be conceived. In the coupled member made of the sensor chips of Claim 3, since the adjacent sensor chips are directly coupled to each other, this adhesive tape may belong to the sensor chips after the coupled member is cut off (if such a portion cut off from sensor chip is produced on adhesive tape, then this portion corresponds to the above-explained holding body, and thus constitutes coupled member made of sensor chips of Claim 4).

There is the coupled member made of a plurality of sensor chips as in the above-described coupled member, wherein
the weak coupling force portion is formed by a structural material of the sensor chip (Claim 5).

In the coupled member made of the sensor chips of Claim 3, preferably, the weak coupling force portion is formed by a structural material of the sensor chips, and Claim 5 corresponds to this preferable embodiment mode. Since the weak coupling force portion is formed by the structural material of the sensor chips, there is no need to use other materials in order to form the week coupling force portion. In this case, the structural material of the sensor chips means such a material which forms the baseplate, thecoverlayer, andalayer (so-called "spacer layer") sandwiched between the base plate and the cover layer, and the like.

There is the coupled member made of the sensor chips as in Claim 3, wherein
the weak coupling force portion includes:
a connection portion for connecting the adjacent sensor chips, and
a cutting portion in which the adj acent sensor chips are cut off (Claim 6).

As methods for forming the weak coupling force portion, the below-mentioned forming methods may be conceived:
1) a method by which adjacent sensor chips are cut off by using a cutter where a portion of cutting blade is killed so as to provide a portion which is cut (cutting portion) and a portion which is not cut (connection portion),
2) a method by which a cutting portion and a connection portion are provided without cutting a portion between adjacent sensor chips,
3) a method (half cut process) by which when adjacent sensor chips are cut, although a cutting blade is entered, the portion between these sensor chips is not completely cut off, but portions along a thickness direction are still coupled to each other,
4) a method by which sensor chips are connected to each other by a connecting member made of a material which is fractured by receiving weak force. Alternatively, these methods may be combined with each other. Claim 6 corresponds to the coupled member made of the a plurality of sensor chips, which is manufactured by the method 1) or 2).

There is the coupled member made of a plurality of sensor chips as in Claim 6, wherein
the coupled member has the connection portions at two or more places for each of the weak coupling force portions (Claim 7).

When easy cutting off characteristics and stability of coupling (cutting off never occurs in case of unwanted opportunity) are considered, in the weak coupling portion constituted by the connection portion and the cutting portion, it is preferable that two or more pieces of the connection portions are provided. However, if a connection portion has a certain length, then the coupling stability may be achieved even one place. Also, when a shallow notch is formed in this connection portion (in such case where notch is formed up to portion along thickness direction), the easy cutting off may be achieved.

There is provided the coupled member made of a plurality of sensor chips as in Claim 3, wherein
the weak coupling force portion is formed by a connecting member which intersects the plurality of sensor chips adjacent to each other (Claim 8).

In this case, the connecting member is formed by such a material which may be fractured by receiving weak force. In other words, Claim 8 corresponds to such an embodiment mode that the weak coupling portion is formed based upon the above-described method 4). As a concrete method belonging to the above-described method, the below-mentioned method may be conceived. That is, a tape is adhered onto a coupled member made of sensor chips formed by employing a large sheet, and only the sensor chips are cut by a cutting blade, or spaces among the sensor chips are cut by the cutting blade so as to be cut off, while this tape is made of such a material which may be fractured by receiving weak force, for example, paper, or a resin film which is easily torn up along a specific direction. Thereafter, before these cut sensor chips are completely separated from each other, such a tape is adhered onto the plurality of sensor chips arranged in the parallel manner.

Also, another method may be conceived. That is, after the sensor chips are cut off as individual sensor chips, the plurality of individual sensor chips are adhered to such a connecting member made of a material which may be fractured by receiving weak force. In accordance with this method, in the case where a certain number of individual sensor chips becomes fail, while only failed sensor chips are removed, a coupled member made of the plurality of sensor chips can be manufactured. As the connecting member of this case, a sheet and a tape which intersect the plurality of sensor chips are exemplified.

There is provided the coupled member made of a plurality of sensor chips in the above-described coupled member, wherein
the sensor chip has a sample introducing port, and
the sample introducing port is opened in a side plane of the sensor chip (Claim 9).

When the sample introducing port is opened in the side plane of the sensor chips, this sample introducing port is covered by a side plane of an adjoining sensor chip, or by the holding body, so that it is possible to avoid that this sample introducing port is touched to be contaminated.

There is provided the coupled member made of a plurality of sensor chips as in the above-described coupled member, wherein
the sensor chip is a biosensor chip (Claim 10).

The sensor chip of the present invention may be suitably employed as, especially, a biosensor chip. The biosensor chip of the present invention may be utilized as, for instance, blood sugar level sensors and urinary sugar level sensors in order to perform home health diagnoses (self-medical cares) capable of self-managing and preventing diabetes. The blood sugar level sensors measure glucose amounts (blood sugar levels) in blood and the urinary sugar level sensors measure glucose amounts in urine. More specifically, in the case where these biosensor chips are folded within a container, or are rounded in the container so as to be provided as a coupled member made of sensor chips to consumers, it is possible to avoid that these biosensor chips are dropped when the biosensor chips are taken from the container, which may give such a great merit with respect to old men and persons having handicaps of eyes, who can hardly handle small sensor chips which are individually separated from each other.

In addition to the above-described coupled member made of the plurality of sensor chips, the present invention may also provide the below-mentioned method for manufacturing a coupled member made of the plurality of sensor chips.

There is provided a method for manufacturing a coupled member made of a plurality of sensor chips which are coupled to each other, the sensor chip including:
a base plate;
a cover layer;
a hollow reaction portion provided between the base plate and the cover layer;
a sensing member provided in the hollow reaction portion;
an output terminal for outputting a signal sensed by the sensing member to the outside; and
a sample introducing port for introducing a sample to the hollow reaction portion,
the method including the steps of:
   forming the plurality of pieces of the hollow reaction portions, the sensing member, and the output terminals between base plate sheets which form the base plate and the cover layer, and thereafter, or at the same time; and
   forming coupling portions for coupling the adjacent sensor chips with each other under cuttable condition in a batch manner (Claim 11).

In this case, the coupling portion for coupling under cuttable condition corresponds to the above-explained weak coupling force portion (also involves such a weak coupling force portion between sensor chip and holding body). In other words, in accordance with this manufacturing method, the structural elements of the respective sensor chips are formed between the large base plate sheets which form the plurality of base plates and the plurality of cover layers respectively, and thereafter, or at the same time, process treatments are carried out in a batch manner by employing a cutter whose a cutting blade is partially killed. As a result, a plurality of pieces of these weak coupling force portions are formed in a batch manner. As a consequence, as to forming of the weak coupling force portions, higher productivity and stability characteristics of forming positions can be achieved. Preferably, the reagents are applied to the hollow reaction portions of the respective sensor chips while the structural elements of the sensor chips are being formed.

There is provided the method for manufacturing the coupled member made of a plurality of sensor chips as in the above-described coupled member, wherein
the plurality of pieces of hollow reaction portions, the sensing member and output terminals are formed between the base plate sheets, and
the adhering portions and the coupling portions are formed in a batch manner, by
a method including the steps of:
forming the plurality of sensing members and output terminals in a parallel manner on one base plate sheet;
stacking sheet layers having one pair of grooves on each other in such a manner that the sensing member is contained in at least one groove, while the one pair of grooves have, as a symmetrical relationship, a folding line which is set as an axis and divides the base plate sheet in two approximately equal parts along the same direction as the parallel direction; and
folding the stacked layer member in two to adhere the sheet layers to each other in such a manner that the sheet layers are located opposite to each other while the folding line is located at a center, or
a method including the steps of:
   folding the base plate sheet in two with setting the folding line to a center, while the sheet layer having a hollow portion is sandwiched in such a manner that the sensing member is contained in the hollow portion; and
   adhering the base plate sheets to the sheet layers (Claim 12).

That is to say, the above-described manufacturing method is such a method for manufacturing the coupled member made of the plurality of sensor chips in which while one sheet of the base plate sheet which constructs the base plate and the cover layer is employed, a plurality of sets of the sensing member, the output terminals, and the hollow reaction portions, or the grooves for forming the hollow reaction portions are formed in the parallel manner. Thereafter, the base plate sheet is folded in two, while the folding line for dividing this base plate sheet by approximately two equal parts is set to the center. In accordance with this method, it is possible to adhere the base plate side to the cover layer side in higher positioning precision.

As the methods for folding in two while the folding line is set to the center, in addition to the method for folding the base plate sheet in two at the position of the folding line, another folding method may be conceived in such a manner that the base plate sheet is folded at positions of two straight lines which are locatedparallel to this folding line and are separated over equal distances from the folding line, and a sectional view thereof becomes a "U-shape."

### Effects of the Invention

From the coupled member made of the plurality of sensor chips, according to the present invention, the respective sensor chips which constitute this coupled member can be readily cut off one by one. Since the positions to be cut off are limited only to such coupling portions that the adjacent sensor chips are coupled to each other under cuttable condition, there is no longer required to determine such a cutting position. As a consequence, while there is no longer required to take time for determining the cutting positions, a higher piece-separating efficiency can be achieved. Also, in this method, the sensor chips are cut off one by one just before these individual sensor chips are stored in the containers, the chip storing operations may be readily carried out, and the failure chips may be easily removed.

Furthermore, the coupled member made of the sensor chips having the above-described structures may be stored in a container by being folded, or rounded without being cut off, and then, the coupled member stored in the container may be provided to consumers. Accordingly, the present invention is capable of avoiding such a problem that when a consumer picks up one necessary chip from the container in order to use sensor chips, all of these stored sensor chips are dropped from the container to be dispersed, and then, some chips are lost, so that the coupled member can be easily handled by the customer.

The sensor chips which are obtained by cutting off the coupled member made of the plurality of sensor chips according to the present invention may be suitably employed as biosensor chips such as blood sugar lever sensors, and may be utilized in home health diagnoses (self-medical cares) and the like. In particular, when these sensor chips are provided to the consumers as the coupled member made of the plurality of sensor chips, there is such a great merit with respect to old men and persons having handicaps of eyes, who can hardly handle small sensor chips which are individually separated from each other.

### Brief Description of the Drawings

[Fig. 1]
   Fig. 1 is a conceptional plan view for indicating an example of a coupled member made of a plurality of sensor chips according to the present invention.
[Fig. 2]
   Fig. 2 is a conceptional plan view for representing another example of a coupled member made of a plurality of sensor chips according to the present invention.

### Best Mode for Carry Out the Invention

Next, a description is made of best modes for carrying out the present invention. The present invention is not limited only to the present embodiment mode, but may be changed to another embodiment mode unless a gist of the present invention is changed.

As an embodiment mode for a coupled member made of a plurality of sensor chips according to the present invention, the below-mentioned coupled member is exemplified. That is, a plurality of sensor chips having rectangular shapes, or the like are coupled to each other in a parallel manner. Each of these sensor chips is provided with a base plate, a cover layer, a hollow reaction portion provided between the base plate and the cover layer, a sensing member provided in the hollow reaction portion, an output terminal, and a sample introducing port. In this case, such a chip arrangement that these sensor chips are coupled to each other in the parallel manner means the following chip arrangement. That is, as shown in Figs. 1 and 2, a large number of sensor chips are arrayed in such a manner that edges of the adjacent sensor chips are contacted to each other by a point contact manner or a line contact manner, or are positioned opposite to each other.

As materials of the base plate and the cover layer, films made of insulating materials are selected. As the insulating materials the below-mentioned insulating materials may be exemplified; ceramics; glass; paper; biodegradation materials (for instance, polylactic acid microbial product polyester etc.); thermoplastic resins such as polyvinyl chloride, polypropropylene, polystyrene, polycarbonate, acrylic resins, polybutylene terephtalate, polyethelene terephtalate (PET); thermosetting resins such as epoxy resins; and plastic materials such as UV hardened materials. In view of mechanical strength, flexibility, easy processing for manufacturing chips, in particular, easy processing for two folded materials, plastic materials such as polyethylene terephtalate may be preferably employed.

A preferable range as to thicknesses of the base plate and the cover layer, whose thicknesses are equal to each other, is varied in response to usage of sensor chips, and is not especially limited. In case of biosensor chips such as blood sugar level sensors, such a thickness range approximately between 100 and 300 µm is preferable.

While a sensor chip which constitutes the coupled member made of the plurality of sensor chips according to the present invention has a hollow reaction portion between a base plate and a cover layer, this hollow reaction portion is formed within a so-called "spacer layer" which is sandwiched between the base plate and the cover layer. The spacer layer is formed by a sheet layer provided in the method for manufacturing the coupled member made of the plurality of sensor chips according to the present invention, or formed by adhering two or more sheets of sheet layers to each other.

The hollow reaction portion corresponds to such a portion that when the sensor chip is used, a sample is introduced, and the introduced sample is chemically reacted. When the sensor chip is a biosensor chip, or the like, a reagent for causing a biochemical reaction of a catalyst, an enzyme, or the like to occur is fixed in this biosensor chip, so that the chemical reaction of a sample is progressed by these members.

For example, in the case where the sensor chip corresponds to a glucose biosensor chip which measures a glucose amount within blood, the below-mentioned layer is formed in this portion; a glucose oxidase layer, a glucose oxidase-electron acceptor (mediator) mixture layer, a glucose oxidase-albumin mixture layer, a glucose oxidase-electron acceptor-albumin mixture layer, or the like.

A sample to be measured, for instance, blood, urine, an aqueous solution sample extracted on a production line, is introduced from a sample introducing port to the above-described hollow reaction portion. Although the sample introducing port may be provided on the base plate, as the previously described preferred embodiment mode, it is preferable to form that the sample introducing port may be opened on at least one side of a long edge of the sensor chip. Alternatively, a plurality of sample introducing ports may be provided, for example, in the case where the sample introducing ports are formed in both side surfaces of the sensor chip and the hollow reaction portion has a straw-shaped structure which are coupled between the sample introducing port and the hollow reaction portion, a sample may be easily acquired, resulting in a preferable sensor structure.

A sensing member is provided in the hollow reaction portion. In this case, the sensing member is constituted by at least two or more pieces of electrodes. Normally, these electrodes are called as an operation electrode and a counter electrode. Further, the sensing member may employ another electrode such as a reference electrode, and other means. The electrodes may achieve such operations that a predetermined voltage is applied to the hollow reaction portion, current values produced by reactions are measured, and the like, while chemical substances contained in samples are detected and quantitative-analyzed based upon signals derived from these electrodes. As the electrodes, carbon electrodes and the like may be employed, and these electrodes may be manufactured on the base plate sheet by executing a screen printing method, and the like.

The above-described electrodes are exposed within the hollow reaction portion. Furthermore, output terminal (lead terminal) portions of these electrodes are formed on the base plate, a spacer layer, or inside the cover layer, otherwise, are formed between these layers. The electrodes are electrically connectable to an external unit of the sensor chip. Thus, a predetermined voltage may be applied or a current value may be measured via the output terminal portions.

The sheet layer which forms the spacer layer may be manufactured by a method for adhering sheets to each other which contain hollow portions and grooves, or another method in which sheet layers are coated by a screen printing method and the coated sheet layers are hardened, if necessary. As a hardening method, a thermal hardening method by heat and a hardening method by UV may be employed. A desirable hardening method may be selected, depending upon sorts of resins to be used. In order to adhere the sheet layers to each other, and adhere the sheet layers on the base plate sheet, an adhesive material, or a gluing agent is employed. These may be applied by using a screen printing method in a batch manner.

An application of a reagent may be carried out before the hollow reaction portion is formed, or after the hollow reaction portion is formed. In such a case that a hollow reaction portion is formed by adhering sheet layers having grooves to each other, normally, it is preferable to apply a reagent after the sheet layers having the grooves are formed before the hollow reaction portion is formed, because the reagent applying work may be easily carried out, and also, positioning of the reagent application may be readily performed.

Referring now to drawings, an example as to a coupled member of the present invention will be described.

Fig. 1 is a conceptional plan view for representing one example of a coupled member made of a plurality of sensor chips according to the present invention. In this example, respective sensor chips 1 which constitute the coupled member made of the sensor chips are arranged in such a manner that short edges 2, 2' of both sides of these sensor chips 1 are ride on one straight line, and long edges 3 of adjacent sensor chips 1 are located opposite to each other, while the adjacent sensor chips 1 are coupled to each other by weak coupling force portions 4.

The weak coupling force portion 4 is constituted by connection portions 5 at two places and a cutting portion 6. Each of the weak coupling force portions 4 is formed as follows. That is, after elements of the respective sensor chips 1 are formed in a parallel manner between two base plate sheets corresponding to the base plate and the cover layer respectively, these elements are press-processed by employing a cutter of which cutting blades at the positions corresponding to the connection portions 5 are killed. Since each of the sensor chips 1 has such a structure that a spacer layer (not shown) is sandwiched between two base plate sheets, the connection portion 5 also has such a structure that a material for constructing the spacer layer is sandwiched between the materials which constitute two base plate sheets.

As the connection portion of the weak coupling force portion 4, instead of the above-described example that the connection portions 5 are provided at two places, a connection portion where a shallow notch is formed may be alternatively provided at one place. In this alternative case, it is preferable that a width (namely, length along long edge 3) of the single connection portion may be made larger.

Fig. 2 is a conceptional plan view for indicating another example as to a coupled member made of a plurality of sensor chips according to the present invention. In this example, respective sensor chips 7 which constitute the coupled member of the plurality of sensor chips are coupled to a holding body 8 by a holding member 9. A base plate and a cover layer of the sensor chip 7, the holding body 8, and the holding member 9 are made of the same material. A cutting portion 10 is present between the sensor chip 7 and the holding body 8. Holding force exerted by the holding member 9 is weak, so that the respective sensor chips 7 can be easily exfoliated from the holding body 8. As a consequence, the adj acent sensor chips 7 can be also cutting away in an easy manner.

## Claims

1. A coupled member made of a plurality of sensor chips, wherein
adj acent sensor chips are coupled to each other under cuttable condition.

2. The coupled member made of a plurality of sensor chips as claimed in claim 1, wherein
the sensor chip comprising:
a base plate;
a cover layer;
a hollow reaction portion provided between the base plate and the cover layer;
a sensing member provided in the hollow reaction portion;
an output terminal for outputting a signal sensed by the sensing member to the outside; and
a sample introducing port for introducing a sample to the hollow reaction portion.

3. The coupled member made of a plurality of sensor chips as claimed in claim 1 or 2, wherein
the adjacent sensor chips are directly coupled to each other by a weak coupling force portion located between the sensor chips.

4. The coupled member made of a plurality of sensor chips as claimed in claim 1 or 2, wherein
the adjacent sensor chips are coupled to each other via a holding body.

5. The coupled member made of a plurality of sensor chips as claimed in claim 3, wherein
the weak coupling force portion is formed by a structural material of the sensor chip.

6. The coupled member made of a plurality of sensor chips as claimed in claim 3 or 5, wherein
the weak coupling force portion includes:
a connection portion for connecting the adjacent sensor chips, and
a cutting portion in which the adj acent sensor chips are cut off.

7. The coupled member made of a plurality of sensor chips as claimed in claim 6, wherein
the coupled member has the connection portions at two or more places for each of the weak coupling force portions.

8. The coupled member made of a plurality of sensor chips as claimed in claim 3, wherein
the weak coupling force portion is formed by a connecting member which intersects the plurality of sensor chips adjacent to each other.

9. The coupled member made of a plurality of sensor chips as claimed in any one of claims 1 to 8, wherein
the sensor chip has a sample introducing port, and
the sample introducing port is opened in a side plane of the sensor chip.

10. The coupled member made of a plurality of sensor chips as claim in any one of claims 1 to 9, wherein
the sensor chip is a biosensor chip.

11. A method for manufacturing a coupled member made of a plurality of sensor chips which are coupled to each other, the sensor chip comprising:
a base plate;
a cover layer;
a hollow reaction portion provided between the base plate and the cover layer;
a sensing member provided in the hollow reaction portion;
an output terminal for outputting a signal sensed by the sensing member to the outside; and
a sample introducing port for introducing a sample to the hollow reaction portion,
the method comprising the steps of:
forming a plurality of pieces of the hollow reaction portions, the sensing member, and the output terminals between base plate sheets which form the base plate and the cover layer, and thereafter, or at the same time, and
forming coupling portions for coupling the adj acent sensor chips with each other under cuttable condition in a batch manner.

12. The method for manufacturing the coupled member made of a plurality of sensor chips as claimed in claim 11, wherein
the plurality of pieces of hollow reaction portions, the sensing members and output terminals are formed between the base plate sheets, and
the adhering portions and the coupling portions are formed in a batch manner, by
a method comprising the steps of:
forming a plurality of sensing member and output terminals in a parallel manner on one base plate sheet;
stacking sheet layers having one pair of grooves on each other in such a manner that the sensing member is contained in at least one groove, while the one pair of grooves have, as a symmetrical relationship, a folding line which is set as an axis and divides the base plate sheet in two approximately equal parts along the same direction as the parallel direction; and
folding the stacked layer member in two to adhere the sheet layers to each other in such a manner that the sheet layers are located opposite to each other while the folding line is located at a center, or
a method comprising the steps of:
folding the base plate sheet in two with setting the folding line to a center, while the sheet layer having a hollow portion is sandwiched in such a manner that the sensing member is contained in the hollow portion; and
adhering the base plate sheets to the sheet layers .
